# EUROPEAN PATENT APPLICATION

(11) **EP 2 182 076 A2**
(43) Date of publication of application: **05.05.2010**
(21) Application number: 09173214.9
(22) Date of filing: 15.10.2009
(51) Int. Cl.: C12Q 1/68

(54) **Analysis of the genetic disposition for epidermolysis bullosa in sheep**

(30) Priority: 15.10.2008 EP 08166726
(71) Applicant: Stiftung Tierärztliche Hochschule Hannover, 30559 Hannover (DE)
(72) Inventor: Distl, Ottmar Prof. Dr. Dr., 30559 Hannover (DE); Mömke, Stefanie Dr., 30173 Hannover (DE); Kerkmann, Andrea, 52156 Monschau (DE)
(74) Representative: Taruttis, Stefan Georg

(57) **Abstract**

The invention provides a method for genetic analysis, which is suitable to predict the individual risk to develop phenotypic JEB, and for the determination of the individual risk for hereditary transmission of an allele causing JEB. The method for genetic analysis can be used on nucleic acid molecules derived from animals, especially sheep. Preferably, the genetic analysis is carried out on total DNA and/or RNA isolated from an individual, e.g. DNA isolated from a blood sample.

The genetic analysis identifies the presence of alleles of genetic markers that have been found to be coupled to JEB. Generally, the analysis comprises the identification of the presence of mutant alleles of genetic markers that have been found to be present in sheep affected by phenotypic JEB, whereas wild-type alleles of the markers have been found to be associated with a non - JEB phenotype or non - JEB predisposition.

SEQUENCE LISTING

<110>  Stiftung Tierärztliche Hochschule Hannover

<120>  Analysis for the genetic disposition for epidermolysis bullosa in
        sheep

<130>  T1009-N-EP

<160>  50

<170>  PatentIn version 3.5

<210>  1
<211>  21
<212>  DNA
<213>  Artificial

<220>
<223>  /note="genomic marker TIHO_EB 1, SEQ ID No 1"

<400>  1
gtttttgcta cgggcattca g                                                21


<210>  2
<211>  21
<212>  DNA
<213>  Artificial

<220>
<223>  /note="genomic marker TIHO_EB 2, SEQ ID No 2"

<400>  2
ctcaggctgg tggaggagca g                                                21


<210>  3
<211>  21
<212>  DNA
<213>  Artificial

<220>
<223>  /note="genomic marker TIHO_EB 3, SEQ ID No 3"

<400>  3
ctgaagacta ttccaagcaa g                                                21


<210>  4
<211>  21
<212>  DNA
<213>  Artificial

<220>
<223>  /note="genomic marker TIHO_EB 4, SEQ ID No 4"

<400>  4
gtgctgctac tgacgcccag a                                                21


<210>  5
<211>  21
<212>  DNA
<213>  Artificial

<220>
<223>  /note="genomic marker TIHO_EB 5, SEQ ID No 5"

<400>  5
tgctgctact gacgcccaga g                                                21

<210>  6
<211>  21
<212>  DNA
<213>  Artificial

<220>
<223>  /note="genomic marker TIHO_EB 6, SEQ ID No 6"

<400>  6
acagcagagc tgagaatgct g                                                21

<210>  7
<211>  21
<212>  DNA
<213>  Artificial

<220>
<223>  /note="genomic marker TIHO_EB 7, SEQ ID No 7"

<400>  7
tggcagtggg gccacctccg c                                                21

<210>  8
<211>  21
<212>  DNA
<213>  Artificial

<220>
<223>  /note="genomic marker TIHO_EB 8, SEQ ID No 8"

<400>  8
agacaagcat cgatggcatt c                                                21

<210>  9
<211>  21
<212>  DNA
<213>  Artificial

<220>
<223>  /note="genomic marker TIHO_EB 9 , SEQ ID No 9"

<400>  9
atttcccaac tggggttctt a                                                21

<210>  10
<211>  22
<212>  DNA
<213>  Artificial

<220>
<223>  /note="genomic marker TIHO_EB 10, SEQ ID No 10"

<400>  10
gtgggatggg ggacatttga ag                                               22

<210>  11
<211>  21
<212>  DNA
<213>  Artificial

<220>
<223>  /note="genomic marker mutTIHO_EB 1, SEQ ID No 11"

<400>  11
gtttttgcta tgggcattca g                                                21


<210>  12
<211>  21
<212>  DNA
<213>  Artificial

<220>
<223>  /note="genomic marker mutTIHO_EB 2, SEQ ID No 12"

<400>  12
ctcaggctgg cggaggagca g                                                21


<210>  13
<211>  21
<212>  DNA
<213>  Artificial

<220>
<223>  /note="genomic marker mutTIHO_EB 3, SEQ ID No 13"

<400>  13
ctgaagacta ctccaagcaa g                                                21


<210>  14
<211>  21
<212>  DNA
<213>  Artificial

<220>
<223>  /note="genomic marker mutTIHO_EB 4, SEQ ID No 14"

<400>  14
gtgctgctac caacgcccag a                                                21


<210>  15
<211>  21
<212>  DNA
<213>  Artificial

<220>
<223>  /note="genomic marker mutTIHO_EB 5, SEQ ID No 15"

<400>  15
tgctgctacc aacgcccaga g                                                21


<210>  16
<211>  21
<212>  DNA
<213>  Artificial

<220>
<223>  /note="genomic marker mutTIHO_EB 6, SEQ ID No 16"

<400>  16
acagcagagc cgagaatgct g                                                21


<210>  17
<211>  21

<212>  DNA
<213>  Artificial

<220>
<223>  /note="genomic marker mutTIHO_EB 7, SEQ ID No 17"

<400>  17
tggcagtggg accacctccg c                                                21


<210>  18
<211>  21
<212>  DNA
<213>  Artificial

<220>
<223>  /note="genomic marker mutTIHO_EB 8, SEQ ID No 18"

<400>  18
agacaagcat agatggcatt c                                                21


<210>  19
<211>  21
<212>  DNA
<213>  Artificial

<220>
<223>  /note="genomic marker mutTIHO_EB 9, SEQ ID No 19"

<400>  19
atttcccaac cggggttctt a                                                21


<210>  20
<211>  21
<212>  DNA
<213>  Artificial

<220>
<223>  /note="genomic marker mutTIHO_EB 10, SEQ ID No 20"

<400>  20
gtgggatggg gacatttgaa g                                                21


<210>  21
<211>  22
<212>  DNA
<213>  Artificial

<220>
<223>  /note="marker specific primer EB1-f , SEQ ID No 21"

<400>  21
aaagtagaaa tgatcagtca cc                                               22


<210>  22
<211>  21
<212>  DNA
<213>  Artificial

<220>
<223>  /note="marker specific primer EB1-r, SEQ ID No 22"

<400>  22
aataggaaat gcagggagtt g                                                21

<210>  23
<211>  19
<212>  DNA
<213>  Artificial

<220>
<223>  /note="marker specific primer EB2-f, SEQ ID No 23"

<400>  23
actgctttcg aaggctcag                                                    19


<210>  24
<211>  18
<212>  DNA
<213>  Artificial

<220>
<223>  /note="marker specific primer EB2-r , SEQ ID No 24"

<400>  24
cgggtccggt agctattc                                                     18


<210>  25
<211>  20
<212>  DNA
<213>  Artificial

<220>
<223>  /note="marker specific primer EB3-f, SEQ ID No 25"

<400>  25
gccaataaca tggagcaact                                                   20


<210>  26
<211>  19
<212>  DNA
<213>  Artificial

<220>
<223>  /note="marker specific primer EB3-r, SEQ ID No 26"

<400>  26
ggaaggactg atcactgac                                                    19


<210>  27
<211>  20
<212>  DNA
<213>  Artificial

<220>
<223>  /note="marker specific primer EB4-f, SEQ ID No 27"

<400>  27
gcaggttgaa gacagaaaag                                                   20


<210>  28
<211>  19
<212>  DNA
<213>  Artificial

<220>
<223>  /note="marker specific primer EB4-r, SEQ ID No 28"

<400>  28
gatcttgccg gcgatattc                                                    19


<210>  29
<211>  20
<212>  DNA
<213>  Artificial

<220>
<223>  /note="marker specific primer EB5-f, SEQ ID No 29"

<400>  29
gcaggttgaa gacagaaaag                                                   20


<210>  30
<211>  19
<212>  DNA
<213>  Artificial

<220>
<223>  /note="marker specific primer EB5-r, SEQ ID No 30"

<400>  30
gatcttgccg gcgatattc                                                    19


<210>  31
<211>  19
<212>  DNA
<213>  Artificial

<220>
<223>  /note="marker specific primer EB6-f, SEQ ID No 31"

<400>  31
gtgattgcag aagcccaga                                                    19


<210>  32
<211>  21
<212>  DNA
<213>  Artificial

<220>
<223>  /note="marker specific primer EB6-r, SEQ ID No 32"

<400>  32
ctattagatg taggatgcta t                                                 21


<210>  33
<211>  20
<212>  DNA
<213>  Artificial

<220>
<223>  /note="marker specific primer EB7-f SEQ ID No 33"

<400>  33
gcctggcagt gtagatgaag                                                   20


<210>  34
<211>  20
<212>  DNA
<213>  Artificial

<220>
<223>  /note="marker specific primer EB7-r, SEQ ID No 34"

<400>  34
tccatctggt ctaccctttg                                                   20


<210>  35
<211>  20
<212>  DNA
<213>  Artificial

<220>
<223>  /note="marker specific primer EB8-f, SEQ ID No 35"

<400>  35
gcctggcagt gtagatgaag                                                   20


<210>  36
<211>  20
<212>  DNA
<213>  Artificial

<220>
<223>  /note="marker specific primer EB8-r, SEQ ID No 36"

<400>  36
tccatctggt ctaccctttg                                                   20


<210>  37
<211>  18
<212>  DNA
<213>  Artificial

<220>
<223>  /note="marker specific primer EB9-f, SEQ ID No 37"

<400>  37
cgagccaaga cccagatc                                                     18


<210>  38
<211>  22
<212>  DNA
<213>  Artificial

<220>
<223>  /note="marker specific primer EB9-r, SEQ ID No 38"

<400>  38
ttactgacca cagcatgact tc                                                22


<210>  39
<211>  18
<212>  DNA
<213>  Artificial

<220>
<223>  /note="marker specific primer EB10-f, SEQ ID No 39"

<400>  39
cgagccaaga cccagatc                                                     18


<210>  40
<211>  22

<212>  DNA
<213>  Artificial

<220>
<223>  /note="marker specific primer EB10-r, SEQ ID No 40"

<400>  40
ttactgacca cagcatgact tc                                                22


<210>  41
<211>  19
<212>  DNA
<213>  Artificial

<220>
<223>  /note="marker TIHO_EB1 amplificate specific internal primer SEQ
       ID No 41"

<400>  41
catcctgcag acagaagtc                                                    19


<210>  42
<211>  20
<212>  DNA
<213>  Artificial

<220>
<223>  /note="marker TIHO_EB2 amplificate specific internal primer SEQ
       ID No 42"

<400>  42
acatctgctg aactgttacc                                                   20


<210>  43
<211>  17
<212>  DNA
<213>  Artificial

<220>
<223>  /note="marker TIHO_EB3 amplificate specific internal primer SEQ
       ID No 43"

<400>  43
cttgcaccac ggagcca                                                      17


<210>  44
<211>  19
<212>  DNA
<213>  Artificial

<220>
<223>  /note="marker TIHO_EB7 amplificate specific internal primer SEQ
       ID No 44"

<400>  44
ggcagcttca gtgttgctc                                                    19


<210>  45
<211>  17
<212>  DNA
<213>  Artificial

<220>
<223>  /note="marker TIHO_EB9 amplificate specific internal primer SEQ

ID No 45"

<400>  45
ccacctccgc tcactgg                                                    17


<210>  46
<211>  17
<212>  DNA
<213>  Artificial

<220>
<223>  /note="marker TIHO_EB10 amplificate specific internal primer SEQ
       ID No 46"

<400>  46
ccacctccgc tcactgg                                                    17


<210>  47
<211>  16
<212>  DNA
<213>  Artificial Sequence

<220>
<223>  /note="genomic marker TIHO_EB 11, SEQ ID No 47"

<400>  47
gaagaatgag agacag                                                     16


<210>  48
<211>  14
<212>  DNA
<213>  Artificial Sequence

<220>
<223>  /note="genomic marker mutTIHO_EB 11, SEQ ID No 48"

<400>  48
gaagaatgag agag                                                       14


<210>  49
<211>  20
<212>  DNA
<213>  Artificial Sequence

<220>
<223>  /note="marker specific primer EB 11-f, SEQ ID No 49"

<400>  49
gagttcaagc gtgtgcaaag                                                 20


<210>  50
<211>  20
<212>  DNA
<213>  Artificial Sequence

<220>
<223>  /note="marker specific primer EB 11-r, SEQ ID No 50"

<400>  50
ggcagcaata ccatgacctt                                                 20

## Description

The present invention relates to a process for analysis of the genetic disposition in individual sheep to develop junctional epidermolysis bullosa (JEB), and to identify individuals carrying the hereditary trait, i.e. an allele coupled to and/or causative for JEB. In accordance with the process for analysis, which preferably is used for the genetic analysis of sheep, the invention also relates to the relevant genetic markers, mutations in which have been found to be characteristic for JEB, and to the use of the relevant genetic markers in the analytical process. Further, the invention relates to oligonucleotides which are specific for DNA or RNA sections containing the relevant genetic markers, e.g. to an allele coupled to JEB, e.g. for use of the oligonucleotides as primers in PCR amplification using genomic DNA of an individual sheep. Further, the invention relates to the oligonucleotides usable as primers for specific amplification of markers coupled to JEB.

The preferred race of sheep for the analytical process is German black headed mutton sheep, its cross-breeds as well as other sheep breeds, as well as Suffolk, Merino, Merino Longwool, East Friesian, and Bavarian Forest sheep, as well as cross-breeds thereof.

The analytical process of the invention is based on the detection of the least one genetic aberration in at least one of genetic markers, which have been found to be indicative of the genetic disposition of the animal itself to develop phenotypic JEB, or to pass on the genetic trait for JEB to offspring. Accordingly, the invention also provides a process for the determination of the individual risk of a sheep to develop phenotypic JEB, as well as a process for determination of the propensity of an individual sheep to pass on the genetic trait for phenotypic JEB to offspring, e.g. in a process for selecting breeding animals.

### State of the art

Today, junctional epidermolysis bullosa (JEB), which is an inherited skin disease known in humans and domestic animals, can only be detected phenotypically. However, diagnosis is often difficult because clinical signs are not very specific. For individuals, which are not phenotypically affected by JEB but which carry an allele for JEB, can only be identified by progeny testing, i.e. indirectly.

It is known that JEB is a lethal genetic trait with monogenic autosomal recessive inheritance. Phenotypically, JEB is a progressive disease in sheep, during which the epidermis gets separated from the dermis resulting in secondary inflammations, and in the case of hoofed animals, in the splitting of the hoof horn. Especially in sheep affected by JEB, standing and walking is painful, and affected lambs develop with retardation in comparison to their healthy twins. Despite therapy, no recovery can be observed, and/or affected lambs die or have to be euthanized for animal welfare reasons. It has been found that JEB is a disrupture within the lamina lucida of the dermoepidermal basement membrane, caused by the failure of the proteins of the dermo - epidermal junction zone. Typically affected regions include the dorsum of the carpal joints, in hoofed animals the hooves, and the oral mucus membranes (tongue, lips).

Pulkkinen et al. in Molecular Medicine 124-135 (1997) describe SNPs that have been identified in single human JEB patients. These known SNPs of GenBank accession No. ZI5008 and ZI5009, e.g. a C-to-A transition at nucleotide position 2740 or position 2805 have a differing location than markers of the present invention, which e.g. in exon 18 of the LAMC2 gene in sheep are further downstream than the SNPs reported previously.

Further, Pulkkinen et al. in Nature Genetics, 293-298 (1994), Aberdam et al. in Nature Genetics 299-304 (1994), Nakano et al. in Hum. Genet. 41-51 (2002), and US2003/0143545 describe JEB-related mutations found in the LAMC2 gene of different species.

### Objects of the invention

In view of the shortcomings of prior art, it is an object of the present invention to provide a genetic test for the analysis of animals, including sheep as well as humans, to determine the presence or absence of alleles coupled to or responsible for JEB, e.g. to determine the genetic predisposition of an individual to pass on an allele causing JEB, and the predisposition of the individual to develop phenotypic JEB.

### General description of the invention

The invention achieves the above-mentioned objects by the features of the claims, especially by providing a method for genetic analysis as well as genetic markers (SNPs) useful in the method, which is suitable to predict the individual risk to develop phenotypic JEB, and for the determination of the individual risk for hereditary transmission of an allele causing JEB. The method for genetic analysis can be used on nucleic acid molecules derived from animals, especially sheep, but also from humans. Preferably, the genetic analysis is carried out on total DNA and/or RNA isolated from an individual, e.g. DNA isolated from a blood sample.

The genetic analysis identifies the presence of alleles of genetic markers that have been found to be coupled to JEB. Generally, the analysis comprises the identification of the presence of mutant alleles of genetic markers that have been found to be present in sheep affected by phenotypic JEB, whereas wild-type alleles of the markers have been found to be associated with a non - JEB phenotype or non - JEB predisposition. In detail, the analytical method of the invention comprises the detection of aberrations in the nucleotide sequence of wild-type markers. Examples of aberrations, also called mutations, which are coupled or causative for the disposition for JEB are given as mutant alleles of the markers

The invention also relates to the use of DNA and/or RNA sections, especially genomic DNA sections, comprising the genetic markers in an analytical process for determination of aberrations in the markers, and to the use of the genetic markers in an analytical process for detecting alleles coupled to JEB, as well as to the use of the oligonucleotides suitable as primers for amplifying a DNA section containing the genetic markers. Preferably, these DNA sections are nucleic acid molecules which are present in multiple copies, e.g. obtained by PCR on the basis of total genomic DNA isolated from the individual to be tested for predisposition for JEB. Preferably, these nucleic acid molecules have between 100 and 659 bp, e.g. obtained by amplification using PCR with pairs of the specific primers described herein. Herein, the term "genetic marker" or "marker" refers to an oligonucleotide having a specific base sequence, comprising both DNA and RNA oligonucleotides. Generally, nucleotide sequences herein are given from 5' to 3'.

As used herein, the wild-type alleles of the markers are not associated with JEB, and, accordingly, the presence of both marker alleles in the wild-type sequence (TIHO_EB 1 to TIHO_EB 11) indicates a genome free from the disposition for JEB, of an individual, whereas mutant sequences of at least one of the markers indicate that the individual tested is a carrier of a genetic factor coupled to a JEB phenotype. Therefore, the presence of an aberration of the base sequence at least one of the markers also indicates the risk of the individual to pass on the hereditary trait for JEB to offspring. Further, at least the presence of homozygous markers indicates the high propensity of the individual to develop phenotypic JEB.

The analytical method identifies alleles of genetic markers which could be shown to be coupled to genetic factors which are contributing to the JEB phenotype, or which are responsible for the JEB phenotype. Accordingly, the invention also provides the genetic markers, aberrations of which from the wild-type sequence are indicative of genetic factors coupled to or responsible for phenotypic JEB, at least when the aberrant marker sequences are present homozygously. Further, specific oligonucleotide sequences are provided that can be used as specific primers in PCR reactions for specific amplification of the DNA fragments containing the genetic markers.

The analytical method is based on the determination of at least one aberration, i.e. at least one mutation, especially a single nucleotide polymorphism (SNP), in at least one of the genetic markers. Preferably, the method of the invention comprises the steps of providing nucleic acids, which preferably is genomic DNA, from an individual, preferably a sheep, e.g. by isolating nucleic acids from a sample of the individual, followed by detection of the presence or absence of an aberration of at least one of the markers TIHO_EB1 to TIHO_EB11. The detection can e.g. be by nucleic acid sequencing or by restriction fragment analysis, which methods are generally known. Preferably the genomic nucleic acids, e.g. genomic DNA, prior to sequence analysis is amplified, e.g. using PCR on a nucleic acid segment containing the marker sequence. Markers which are relevant for the genetic disposition for JEB are given in table 1 below, wherein the wild-type sequence of the marker indicates the allele of the genetic marker not contributing to or causing JEB, whereas aberrations in the genetic markers indicate a contributing factor to the genetic disposition for JEB.

In the mutant marker sequences given, exemplary SNPs are indicated, which could be shown to be coupled to JEB least in sheep, including German black headed mutton sheep, its cross-breds, and also in other sheep breeds. Accordingly, it is preferred that the nucleic acid used in the methods of the invention is obtained from a bovid, especially from a sheep.

For the purposes of this invention, the LAMC2 gene preferably has the sheep sequence, e.g. as accessible under the accession number FM872310.1. The markers of the invention, including their mutant alleles, are preferably contained in the sequence accessible under accession number FM872310.1, which is hereby incorporated as part of the description, most preferably available at NM_001142358 (e.g. at NCBI), e.g. as the origin of the markers and/or of the mutant marker alleles.

Each of the wild-type marker sequences, which are termed TIHO_EB 1 (SEQ ID No. 1), TIHO_EB 2 (SEQ ID No. 2), TIHO_EB 3 (SEQ ID No. 3), TIHO_EB 4 (SEQ ID No. 4), TIHO_EB 5 (SEQ ID No. 5), TIHO_EB 6 (SEQ ID No. 6), TIHO_EB 7 (SEQ ID No. 7), TIHO_EB 8 (SEQ ID No. 8), TIHO_EB 9 (SEQ ID No. 9), TIHO_EB 10 (SEQ ID No. 10), and TIHO_EB11 (SEQ ID No. 47) has been found not to be coupled with JEB. In contrast, mutant alleles of these wild-type markers could be identified by an SNP, which mutant variants have been found to be indicative of the genetic disposition for JEB. The following mutant sequences (mutTIHO_EB) of each of the markers (TIHO_EB) are examples for marker sequences coupled to the genetic predisposition for JEB: mutTIHO_EB 1 (SEQ ID No. 11), mutTIHO_EB 2 (SEQ ID No. 12), mutTIHO_EB 3 (SEQ ID No. 13), mutTIHO_EB 4 (SEQ ID No. 14), mutTIHO_EB 5 (SEQ ID No. 15), mutTIHO_EB 6 (SEQ ID No. 16), mutTIHO_EB 7 (SEQ ID No. 17), mutTIHO_EB 8 (SEQ ID No. 18), mutTIHO_EB 9 (SEQ ID No. 19), mutTIHO_EB 10 (SEQ ID No. 20), and mutTIHO_EB 11 (SEQ ID No. 48).
As all the genetic markers, mutations in which indicate a predisposition for JEB, are localised in the *laminin gamma 2* (*LAMC2*) gene (the sequence of which is e.g. accessible under accession number FM872310.1), the present invention also relates to a method for analysis of an individual for the genetic disposition for JEB, comprising the sequence analysis of the *LAMC2* gene, including its 3' untranslated region.

Accordingly, the invention also relates to the use of an isolated DNA sequence, the isolated sequence comprising the *LAMC2* gene or sections thereof, especially its 3 'untranslated region, its exon sequences, and/or its intron sequences, obtained from an individual, for analysis of aberration in the *LAMC2* gene in respect of mutations coupled to or responsible for JEB. Preferred exon sequences consist of exon 5, exon 13, exon 16, exon 17, exon 18, exon 20, exon 22, and/or exon 23; preferred untranslated sequences consist of the 3' untranslated region, intron 4, intron 5, and intron 13, of the *LAMC2* gene.

The occurrence of the correlation of the mutation of the markers, especially of each of TIHO_EB11 and TIHO_EB7, was verified in Suffolk, Merino, Merino Longwool, East Friesian, and Bavarian Forest sheep. Specifically, a sample of 176 adult healthy Suffolk sheep was genotyped for TIHO_EB7. The penetrance was found to 95.45%. In the other sheep breeds, individuals with homozygous genotype were not ascertained. The percentage of animals heterozygous in respect of the marker TIHO_EB7 was 13.14% based on genotyping results of adult and healthy individuals, tested on 14 healthy Merino, 37 healthy Merino Longwool, 50 healthy East Friesian, and 10 healthy Bavarian Forest sheep.

An overview of the markers and their wild-type sequences and exemplary mutant sequences which are coupled to JEB is given in table 1.

**Table 1: Marker sequences for non - JEB (wild-type) and JEB - coupled mutant alleles**

| marker wild-type sequence SEQ ID No. | marker mutant sequence SEQ ID No. | JEB affected | JEB carri er | Control | location |
|---|---|---|---|---|---|
| TIHO_EB1 GTTTTTGCTACGGGCA TTCAG SEQ ID No. 1 | mutTIHO_EB1 GTTTTTGCTATGGGC ATTCAG SEQ ID No. 11 | T/T | C/T | C/C | exon 5 |
| TIHO_EB2 CTCAGGCTGGTGGAG GAGCAG SEQ ID No. 2 | mutTIHO_EB2 CTCAGGCTGGCGGA GGAGCAG SEQ ID No. 12 | C/C | C/T | T/T | exon 13 |
| TIHO_EB3 CTGAAGACTATTCCA AGCAAG SEQ ID No. 3 | mutTIHO_EB3 CTGAAGACTACTCCA AGCAAG SEQ ID No. 13 | C/C | C/T | T/T | exon 16 |
| TIHO_EB4 GTGCTGCTACTGACG CCCAGA SEQ ID No. 4 | mutTIHO_EB4 GTGCTGCTACCAACG CCCAGA SEQ ID No. 14 | C/C | C/T | T/T | exon 20 |
| TIHO_EB5 TGCTGCTACTGACGC CCAGAG SEQ ID No. 5 | mutTIHO_EB5 TGCTGCTACCAACGC CCAGAG SEQ ID No. 15 | A/A | A/G | G/G | exon 20 |
| TIHO_EB6 ACAGCAGAGCTGAGA ATGCTG SEQ ID No. 6 | mutTIHO_EB6 ACAGCAGAGCCGAG AATGCTG SEQ ID No. 16 | C/C | C/T | T/T | exon 22 |
| TIHO_EB7 TGGCAGTGGGGCCAC CTCCGC SEQ ID No.7 | mutTIHO_EB7 TGGCAGTGGGACCA CCTCCGC SEQ ID No. 17 | A/A | A/G | G/G | exon 23 |
| TIHO_EB8 AGACAAGCATCGATG GCATTC SEQ ID No.8 | mutTIHO_EB8 AGACAAGCATAGAT GGCATTC SEQ ID No. 18 | A/A | C/A | C/C | exon 23 |
| TIHO_EB9 ATTTCCCAACTGGGG TTCTTA SEQ ID No.9 | mutTIHO_EB9 ATTTCCCAACCGGGG TTCTTA SEQ ID No. 19 | C/C | C/T | T/T | 3' UTR |
| TIHO_EB10 GTGGGATGGGGGACA TTTGAAG SEQ ID No.10 | mutTIHO_EB10 GTGGGATGGGG:ACA TTTGAAG SEQ ID No. 20 | delG/ delG | delG /G | G/G | 3' UTR |
| TIHO_EB11 GAAGAATGAGAGACA G SEO ID No. 47 | mutTIHO_EB11 GAAGAATGAGAGA__ G SEO ID No. 48 | delCA/ delCA | delC A/C A | CA/ CA | exon 18 |

Table 1 shows sequences of the wild-type sequence and of exemplary mutant marker sequences. In the sequences, the nucleotide that is affected by the aberration within a mutant marker sequence is indicated by underlining. Further, table 1 gives the allelic nucleotide combinations identified in individuals phenotypically affected by JEB, the heterozygous allelic combinations for JEB carriers, which usually are phenotypically not affected by JEB, and of genetically free, homozygous allelic combinations as identified in non-affected animals (Control) of the nucleotides affected by the aberration. The exemplary SNPs contained in the exemplary mutant marker sequences are indicated as single base exchanges from the wild-type nucleotide to the mutant nucleotide, and as a deletion of a G for the mutant sequence of TIHO_EB 10. Table 2 also indicates the location of the SNPs in the *LAMC2* gene. As the sections of the genomic DNA specifically amplified by these primers have been identified as regions of the *LAMC2* gene. The respective amplified regions of the *LAMC2* gene are indicated in table 3. Preferably, the analysis of the marker sequences is based on DNA amplification products produced by PCR on a DNA sample isolated from an individual, preferably the sample comprising or consisting of total genomic DNA.

The effect of the SNP in the mutant marker sequence on the amino acid sequence of the relevant protein was identified by comparison of nucleotide sequences, in that the SNPs of mutTIHO_EB1, mutTIHO_EB2, mutTIHO_EB3, mutTIHO_EB4, mutTIHO_EB6, mutTIHO_EB8, mutTIHO_EB9, and mutTIHO_EB10 have no effect on the amino acid sequence, i.e. are silent, whereas mutTIHO_EB5 causes an amino acid exchange Asp1001Asn, and mutTIHO_EB7 causes an amino acid exchange Gly1152Asp in the LAMC2 protein. The preferred marker for use in the analytical method is TIHO_EB11, a mutation of which is believed to be indicative of the causative mutation for JEB in sheep. The mutation identified in TIHO_EB11 (submitted to EMBL accession No. FM872310:c.2746delCA) is a deletion of two base pairs, localized within exon 18 of the ovine *LAMC2* gene. This mutation of TIHO_EB11 leads to a frameshift and a premature stop codon 13 bases downstream of the mutation (Table 1). In addition, incorrect splicing of the exons 18 and 19 of *LAMC2* was shown to be coupled to the presence of the mutant allele of TIHO_EB11 (p.Q916EfsXl3) as well as its mutant allele show a complete co-segregation with the affection status of JEB in an exemplary test 21 affected BHM lambs and 719 unaffected BHM sheep. In a further 337 healthy sheep of other sheep breeds this mutation of TIHO_EB11 was not present. The distribution of the defective allele of TIHO_EB11 in the BHM sheep population was at 0.9%.

It has been found that all markers are contained in the ovine *LAMC2* gene, namely TIHO_EB 1, TIHO_EB 2, TIHO_EB 3, TIHO_EB 4, TIHO_EB 5, TIHO_EB 6, and TIHO_EB 8 in exons, TIHO_EB 7 and TIHO_EB11 in exon 23, as well as TIHO_EB 9 and TIHO_EB 10 in untranslated regions of *LAMC2,* namely in the 3' untranslated region (3' UTR).

Accordingly, the invention also relates to a process for breeding animals, e.g. hoofed animals, preferably ungulates and especially sheep, the process including the step of analysing the genotypes of an individual animal in respect of aberrations in a DNA section including the *LAMC2* gene, preferably in a DNA section including or consisting of exon 5, exon 13, exon 16, exon 20, exon 22, exon 23, and/or in the 3' UTR, especially in a DNA section including at least one of the markers TIHO_EB to TIHO_EB 11 by a method according to the invention.

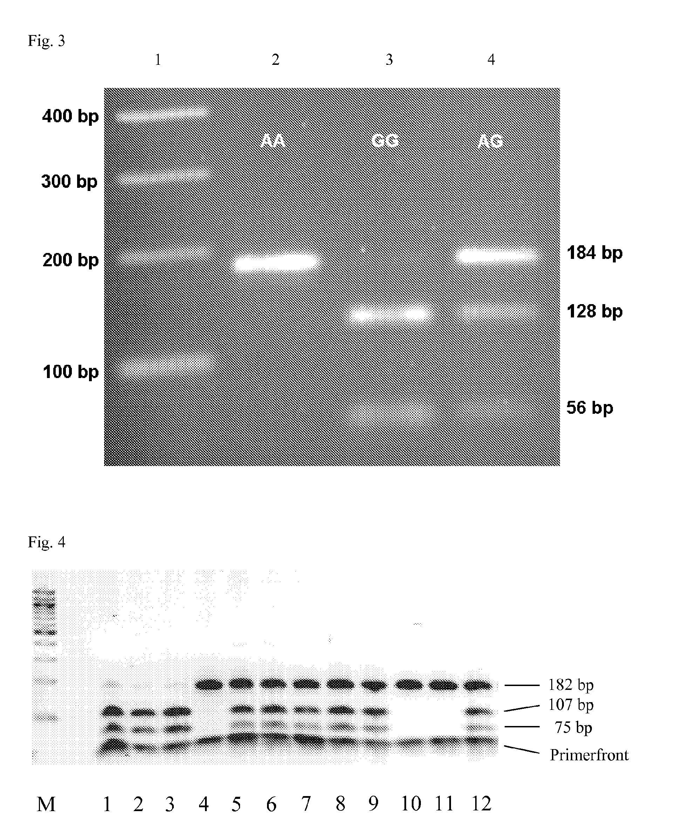

The genetic test for JEB, preferably identifying TIHO_EB11, can be conducted by enzymatic digestion, or sequencing of PCR products, or product size separation on polyacrylamide gels, or using a Taqman® allelic discrimination assay. Preferably, for analysis of mutations of TIHO_EB11, the analysis comprises the enzymatic digestion of a DNA fragment containing TIHO_EB11 or its mutant allele, the fragment preferably generated by PCR, with the MnlI enzyme. Using the specific primers indicated herein for generating a DNA fragement containing the TIHO_EB11 or its mutant allele on the basis of genomic DNA, a PCR product consisting of 182 bp is generated and is cut by MnlI if the mutated form of the sequence is present. The restriction takes place at a temperature of 37°C and requires buffer NEB2 as well as bovine serum albumine. In homozygous wildtype status fragment length is 182 bp, in one mutated status 75 bp and 107 bp and in heterozygous status 75 bp, 107 bp, and 182 bp (Fig. 4). Primers and PCR conditions are given in Tables 2 and 3. Therefore, a diagnostic method using this marker, optionally only using analysis of TIHO_EB11 or of its mutant alleles, is preferable for detecting JEB in sheep, and for a process for breeding comprising the step of estimating the individual risk of a sheep to develop JEB.

Statistic analysis could show that the other markers were in linkage disequilibrium with TIHO_EB11. Accordingly, the analytical method also relates to the detection of mutations in markers TIHO_EB1 to TIHO_EB10, most preferably in addition to analysis of mutations in TIHO_EB11. Preferably, the method comprises the detection of mutations in TIHO_EB7, as this marker has been found to be closely coupled to JEB. TIHO_EB7 can be analysed in the method of the invention in the alternative to TIHO_EB11, but preferably in addition to TIHO_EB11.

Further, it was found that TIHO_EB 9 and TIHO_EB 10 are in linkage disequilibrium to TIHO_EB 7. It could be shown that all markers and their mutant sequences, respectively, were indicative of the absence of presence, respectively, of the genetic predisposition for JEB. Mutant sequences of all markers showed a recessive relationship of the genotype to phenotypic JEB. Accordingly, at least one of the markers can be used for detecting individuals affected by JEB, or carrying the genetic trait responsible for JEB.

It is preferred to analyze at least one, preferably two or more, more preferably at least three, most preferably all of the genetic markers for JEB, most preferably including TIHO_EB 7, to increase the confidence of the estimation of the risk for JEB in an individual, as well as the hereditary contribution of the individual to its offspring to develop JEB or carry the genetic predisposition to further pass on a genotype affected by JEB.

The genetic analysis of the invention allows a prediction of the probability of a male or female individual to pass on the genetic trait for JEB to its offspring, which is of particular relevance for use in animal breeding, e.g. for the selection of future sires, especially for breeding sires and dams, because their genetic predisposition to pass on an allele predisposing or causing JEB to offspring can be determined prior to mating, e.g. when selecting a breeding animal.

### Detailed description of the invention

The invention is now described in greater detail with reference to the figures, showing in
- Figure 1 a graphical representation of the analysis of the linkage disequilibrium among markers TIHO_EB1 to TIHO_EB 10,
- Figure 2 shows the result of a gelelectrophoretic analysis of a PCR amplification of a product containing the marker TIHO_EB 7, with a mutation specific restriction reaction for 4 individual sheep,
- Figure 3 shows the gelelectrophoretic analysis of PCR amplification products containing the marker TIHO_EB 5, with mutation specific restriction for 3 individual sheep, and
- Figure 4 shows the gelelectrophoretic analysis of PCR amplification products containing the marker TIHO_EB11 following restriction with *Mnl*I*.*

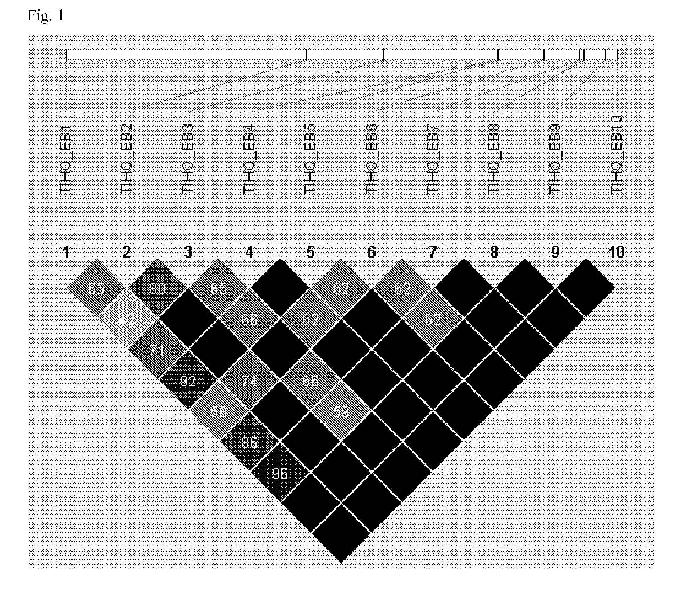

The markers were identified by analysis of about 600 German black headed mutton sheep and its cross-breds and verified in the other sheep breeds mentioned above. For estimating the genetic predisposition of an individual to be affected by phenotypic JEB, or its propensity to develop phenotypic JEB, or to pass on the genetic disposition for JEB to offspring, at least one marker from the group comprising or consisting of markers TIHO_EB 1, TIHO_EB 2, TIHO_EB 3, TIHO_EB 4, TIHO_EB 5, TIHO_EB 6, TIHO_EB 7, TIHO_EB 8, TIHO_EB 9, TIHO_EB 10, and TIHO_EB 11 is analysed for an aberration. Most preferably, marker TIHO_EB 11 is included in the analysis, because its mutant allele indicates a high risk for JEB. In the alternative to TIHO_EB 11, preferably in addition to TIHO_EB 11, marker TIHO_EB 7 is analysed for an aberration, especially in individuals carrying the mutTIHO_EB 11 and/or mutTIHO_EB 7 in both alleles, i.e. in homozygous mutant alleles of mutTIHO_EB 11 and/or TIHO_EB 7. A graphic representation of the linkage disequilibrium of the markers is shown in Figure 1, wherein black boxes indicate linkage disequilibrium coefficients (r²) of 1 and numbers in the boxes indicate r² x 100 with r being the linkage disequilibrium coefficient. The open bar gives relative linkages of the markers.

Preferably, at least one of the markers is analysed for the presence of a mutation, including preferably at least TIHO_EB 11, in the genome of an individual. It is preferred that the analysis includes at least two markers, e.g. TIHO EB 11 and TIHO_EB 7. In addition to analysis of TIHO EB 11 and/or TIHO_EB 7, the method can comprise the analysis of one or more markers in addition to TIHO EB 11 and/or TIHO_EB 7, preferably all markers from TIHO_EB 1 to TIHO_EB 11, for inferring the individual risk for phenotypic JEB, and/or for inferring the propensity to pass on the genetic trait for JEB to offspring. Most preferably, all markers are analysed for the presence of mutations in an individual.

For analysis of the markers for identification of mutant sequences, especially SNPs, sequencing of the marker can be used, or an electrophoretic size separation of DNA sections having sizes which specifically indicate the presence or absence of aberrations in a marker, e.g. using in the analysis restriction fragments of extension products of two specific oligonucleotide primers, of which preferably at least one is labelled, generated by a DNA polymerase, e.g. in PCR.

Further, genomic DNA, preferably amplification products generated from genomic DNA of an individual by PCR of a DNA section containing a marker are further analysed by restriction using a restriction enzyme, the activity of which is dependent on the presence or absence of a mutation in the marker, e.g. RFLP. Specific primers, which are preferably used pairwise as indicated below, are given in table 2 for specific amplification of DNA sections comprising the marker. AT is the preferred annealing temperature for PCR.

**Table 2: Primers for pairwise use for specific amplification of sections of nucleic acid sequences from genomic DNA in a PCR reaction.**

| Marker | Amplified regions of *LAMC2* | forward primer (5' > 3') | backward primer (5' > 3') | AT (°C) | size (bp) |
|---|---|---|---|---|---|
| TIHO_EB1 | exon 5, parts of intron 4 and intron 5 | EB1-f (SEQ ID No. 21) AAAGTAGAAATGA TCAGTCACC | EB1-r (SEQ ID No. 22) AATAGGAAATGC AGGGAGTTG | 60 | 521 |
| TIHO_EB2 | parts of exon 13 and exon 14, intron 13 | EB2-f (SEQ ID No.23) ACTGCTTTCGAAGG CTCAG | EB2-r (SEQ ID No. 24) CGGGTCCGGTAG CTATTC | 58 | 479 |
| TIHO_EB3 | parts of exon 16 and exon 17, intron 16 | EB3-f (SEQ ID No. 25) GCCAATAACATGGA GCAACT | EB3-r (SEQ ID No. 26) GGAAGGACTGAT CACTGAC | 58 | appro x. 800 |
| TIHO_EB4 | parts of exon 20 | EB4-f (SEQ ID No. 27) GCAGGTTGAAGAC AGAAAAG | EB4-r (SEQ ID No. 28) GATCTTGCCGGC GATATTC | 58 | 184 |
| TIHO_EB5 | parts of exon 20 | EB5-f (SEQ ID No. 29) GCAGGTTGAAGAC AGAAAAG | EB5-r (SEQ ID No. 30) GATCTTGCCGGC GATATTC | 58 | 184 |
| TIHO_EB6 | exon 22 | EB6-f (SEQ ID No. 31) GTGATTGCAGAAGC CCAGA | EB6-r (SEQ ID No. 32) CTATTAGATGTA GGATGCTAT | 58 | 100 |
| TIHO_EB7 | parts of exon 23 and parts of 3'ÙTR | EB7-f (SEQ ID No. 33) GCCTGGCAGTGTAG ATGAAG | EB7-r (SEQ ID No. 34) TCCATCTGGTCT ACCCTTTG | 58 | 527 |
| TIHO_EB8 | parts of exon 23 and parts of 3'ÙTR | EB8-f (SEQ ID No. 35) GCCTGGCAGTGTAG ATGAAG | EB8-r (SEQ ID No. 36) TCCATCTGGTCT ACCCTTTG | 58 | 527 |
| TIHO_EB9 | parts of exon 23 and parts of 3'ÙTR | EB9-f (SEQ ID No. 37) CGAGCCAAGACCC AGATC | EB9-r (SEQ ID No. 38) TTACTGACCACA GCATGACTTC | 58 | 659 |
| TIHO_EB 10 | parts of exon 23 and parts of 3'ÙTR | EB10-f (SEQ ID No. 39) CGAGCCAAGACCC AGATC | EB10-r (SEQ ID No. 40) TTACTGACCACA GCATGACTTC | 58 | 659 |
| TIHO_EB 11 | exon 18 | EB11-f (SEQ ID No. 49) GAGTTCAAGCGTGT GCAAAG | EB11-r (SEQ ID No. 50) GGCAGCAATACC ATGACCTT | 60 | 182 |

In table 2, oligonucleotides for specific amplification of a DNA section from total genomic DNA are given, which DNA section contains a marker. In accordance with the markers specifically contained in the amplification products, mutant marker sequences are contained in the amplification product, dependent on the genomic allelic sequences.

In table 2, oligonucleotide primers are named in accordance with the respective TIHO_EB marker numbering, which is specifically amplified as part of the PCR product, as EB-f including the number of the TIHO_EB marker, with "f" indicating a forward-primer, and EB-r , with "r" indicating the reverse-primer, which are preferably used pairwise with the same numbers, e.g. EB1-fpaired with EB1-r.

For detection of amplification products, as well as for sequencing and/or for electrophoretic size separation, it is preferred that at least one labelled (IRD) oligonucleotide is used in PCR, e.g. for amplification of a DNA section comprising one of the markers, and/or in a PCR sequencing reaction using ddNTPs. For sequencing, a LI-COR 400 automatic sequencer LI-COR, Lincoln, USA) was used. As the amplificates containing markers as generated with the oligonucleotide primers of table 2 span exon regions and untranslated regions of the *LAMC2* gene, use of the *LAMC2* gene, especially of its exons 5, 13, 16, 17, 20, and 22, and/or of its introns 4, 5, 13, 16, and of the 3' untranslated region for the analytical method is preferred. Exemplary sequencing primers for identification of wild-type and/or mutant marker sequences in DNA sections containing the markers are given in following table 3, which primers hybridize to genomic DNA sections containing the primers given in table 2, which primers are located upstream and downstream of each marker, respectively. Accordingly, the analytical method preferably comprises the isolation of a genomic DNA section comprising at least one marker, more preferably the amplification of a genomic DNA section comprising at least one marker, e.g. by PCR with the primers of table 2. Preferably, the DNA sections comprise the sequences of the oligonucleotide primers given in table 2, e.g. as PCR amplification products generated by these primers on total genomic DNA. As a sequencing primer, one of the primers of table 2 for a DNA fragment containing the marker can be used in the alternative. For sequencing, it is preferred to use fluorescence labelled primers, e.g. for use in automatic detection of a fluorescence labelled sequencing primer.

**Table 3: Exemplary analysis of PCR products for the detection of a mutation in one of the markers TIHO_EB 1 to TIHO_EB 10**

| Markername | Internal primers for sequencing |
|---|---|
| TIHO_EB1 | CATCCTGCAGACAGAAGTC (reverse primer, SEQ ID No. 41) |
| TIHO_EB2 | ACATCTGCTGAACTGTTACC (reverse primer, SEQ ID No. 42) |
| TIHO_EB3 | CTTGCACCACGGAGCCA (reverse primer, SEQ ID No. 43) |
| TIHO_EB7 | GGCAGCTTCAGTGTTGCTC (reverse primer, SEQ ID No. 44) |
| TIHO_EB8 | GGCAGCTTCAGTGTTGCTC (reverse primer, SEQ ID No. 44) |
| TIHO_EB9 | CCACCTCCGCTCACTGG (forward primer, SEQ ID No. 45) |
| TIHO_EB10 | CCACCTCCGCTCACTGG (forward primer, SEQ ID No. 46) |

Preferred reaction conditions for PCR amplification using these nucleotides are given in table 4.

**Table 4: PCR conditions. The PCR cycle is repeated 37 times and contains steps 2 to 4.**

| | PCR (restriction and MegaBACE) | | |
|---|---|---|---|
| Step 1 | 94 °C | 4:00 | |
| Step 2 | 94 °C | 0:30 | Cycle start |
| Step 3 | AT (of table 1) | 1:00 | |
| Step 4 | 72 °C | 1:20 | Cycle end, 37 x |
| Step 5 | 72 °C | 5:00 | |
| Step 6 | 4 °C | 10:00 | |

The marker, TIHO_EB 7, was found to the localised in exon 23 of the *LAMC2* gene. An exemplary mutation in TIHO_EB 7, presently given as mutTIHO_EB 7, is a transposition of G to A. The clinic and pathologic examination of phenotypically JEB affected lambs of three different farms (a total 21 lambs of the breed German black headed mutton and its cross-breds) were homozygous for the mutant genotypic SNPs A/A (mutTIHO_EB 7 / mutTIHO_EB 7). Accordingly, the analytical method of the invention preferably includes genotyping individuals in respect of mutations in the marker TIHO_EB 7. The occurrence of the correlation of mutations in TIHO_EB 7, especially of mutTIHO_EB 7, 644 healthy unrelated adult sheep of the population of German black headed mutton were genotyped for TIHO_EB 7. Of the sample, 57.76% were homozygous wild-type (G/G, TIHO_EB 7/ TIHO_EB 7)), 37.11% were heterozygous (A/G mutTIHO_EB 7/ TIHO_EB 7)), and only 5.12% were for homozygous mutant, i.e. of the JEB susceptible genotype A/A. On the basis of these figures, a penetrance, i.e. the probability of affection for an individual having the susceptible genotype was calculated to 94.88% for the mutant marker, susceptible genotypic SNP A/A (mutTIHO_EB 7/ mutTIHO_EB 7). Therefore, the marker TIHO_EB 7 is preferably used in a diagnostic method for detecting the propensity of sheep for JEB, both to identify carriers of JEB - related alleles, and to estimate the individual risk to develop phenotypic JEB.

For marker TIHO_EB 7, the χ² test resulted in statistics for the distribution of genotype χ² phenotype 209.25, alleles (χ² allele 111.04), and the trend in alleles (χ² trend 105.79), and their corresponding error probabilities (genotype, allele, and trend below 10⁻⁵).

### Example: Analysis of DNA sections containing markers by RFLP

In this example, gel-electrophoretic analyses are shown for DNA sections containing markers, which are PCR amplificates that were generated on total genomic DNA obtained from different sheep with or without phenotypic JEB, using the primers indicated in table 2 for amplifying DNA fragments containing the respective markers, followed by a restriction reaction, which is specific for a mutation in the marker sequence.

For restriction fragment length polymorphism (RFLP), a section of genomic DNA of individual sheep was amplified by PCR using the primers as indicated in table 3, followed by restriction of the PCR product.

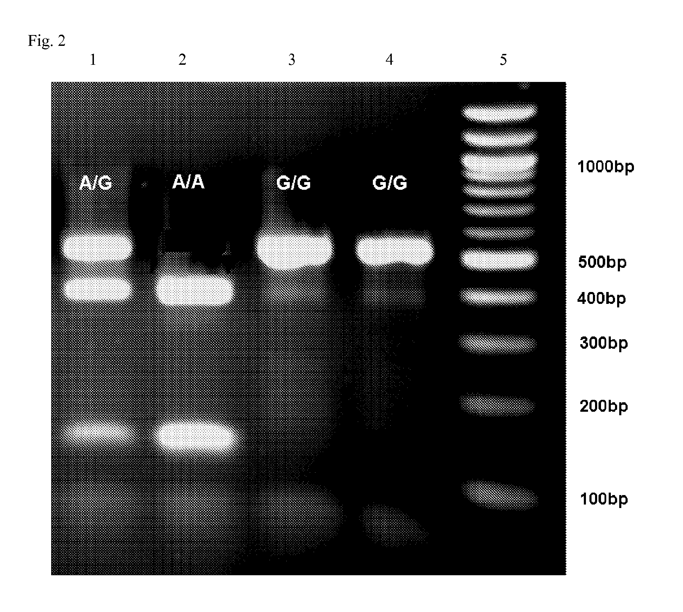

Figure 2 shows the gel-electrophoretic size separation of fragments generated by restriction of PCR amplification products using EB7-f and EB7-r as oligonucleotide primers on total genomic DNA. The amplification products were subjected to restriction with the restriction enzyme AvaII. Wild-type alleles (TIHO_EB 7) are not restricted by AvaII, and homozygous individuals in lanes 3 and 4 (G/G of TIHO_EB 7 / TIHO_EB 7) show one band of 527 bp (lane 4) as compared to the size standards in lane 5. The individual having the genotype A/A shows two bands of 129 bp and 398 bp (lane 2) of the mutant marker (mutTIHO_EB 7 / mutTIHO_EB 7). The heterozygous individual (lane 1) shows three bands of 129 bp, 398 bp, and 527 bp (mutTIHO_EB 7 / TIHO_EB 7).

Figure 3 shows the gel-electrophoretic separation of an RFLP analysis for TIHO_EB 5 using a restriction enzyme BsaHI on a PCR amplificate obtained with EB5-F and EB5-r on total genomic DNA. In comparison to size standards in lane 1, lane 2 shows the result for an individual which is homozygous for the mutant marker sequence (A/A of mutTIHO_EB 5 / mutTIHO_EB 5), giving one band of 184 bp. Lane 3 shows an individual that is homozygous for the wild-type marker sequence TIHO_EB 5 (G/G, giving bands of 128 bp and 56 bp). Lane 4 shows the result for a heterozygous individual (A/G), exhibiting 3 bands of 184 bp, 128 bp and 56 bp.

Figure 4 shows an examplary genetic test in 12 BHM sheep for TIHO_EB11, using restriction by MnII on PCR amplification products generated from chromosomal DNA using the primers EB11-fand EB11-r. In healthy sheep have been found to be homozygous for the wild-type, indicated by a fragment length is 182 bp, in sheep affected by JEB two fragments of 75 bp and 107 bp are present, indicating the mutant allele of the marker homozygously, and in samples from carrier animals, three fragments of 75 bp, 107 bp, and 182 bp are visible, indicating heterozygosity of the wild-type and mutant alleles of the marker TIHO_EB11. In this example, the animals shown in lanes 1, 2, and 3 are affected by JEB, animals in lanes 4, 10, and 11 are unaffected, and animals in lanes 5 to 9 and 12 are carriers of JEB.

These examples show that mutations in the markers of the invention can be detected in total genomic DNA, and that the genotypes of individuals for alleles predisposing for JEB can be analysed by the detection of aberrations in at least one of the markers, and even by an analytical method consisting of the detection of aberrations of TIHO_EB11, which aberration preferably has a sequence according to mutTIHO_EB11.

## Claims

1. Process for the analysis of the genetic disposition of an individual for JEB,
**characterized by** the detection of at least one aberration in the marker TIHO_EB11 (SEQ ID No. 47).

2. Process for the analysis of the genetic disposition of an individual for JEB,
**characterized by** the detection of at least one aberration in the marker TIHO_EB7 (SEQ ID No. 33).

3. Process according to claim 1 or 2, **characterized by** the additional detection of an aberration in at least one of the markers contained in the group consisting of TIHO_EB 1 (SEQ ID No. 1), TIHO_EB 2 (SEQ ID No. 2), TIHO_EB 3 (SEQ ID No. 3), TIHO_EB 4 (SEQ ID No. 4), TIHO_EB 5 (SEQ ID No. 5), TIHO_EB 6 (SEQ ID No. 6), TIHO_EB 7 (SEQ ID No. 7), TIHO_EB 8 (SEQ ID No. 8), TIHO_EB 9 (SEQ ID No. 9), and/or TIHO_EB 10 (SEQ ID No. 10).

4. Process according to claim 1, **characterized in that** the aberration of the marker TIHO_EB11 (SEQ ID No. 47) is mutTIHO_EB11 (SEQ ID No. 48).

5. Process according to one of the preceding claims, **characterized in that** the aberration is contained in the group consisting of DNA sections comprising
EB1-f (SEQ ID No. 21) and EB1-r (SEQ ID No. 22),
EB2-f (SEQ ID No. 23) and EB2-r (SEQ ID No. 24),
EB3-f (SEQ ID No. 25) and EB3-r (SEQ ID No. 26),
EB4-f (SEQ ID No. 27) and EB4-r (SEQ ID No. 28),
EB5-f (SEQ ID No. 29) and EB5-r (SEQ ID No. 30),
EB6-f (SEQ ID No. 31) and EB6-r (SEQ ID No. 32),
EB7-f (SEQ ID No. 33) and EB7-r (SEQ ID No. 34),
EB8-f (SEQ ID No. 35) and EB8-r (SEQ ID No. 36),
EB9-f (SEQ ID No. 37) and EB9-r (SEQ ID No. 38),
EB10-f (SEQ ID No. 39) and EB10-r (SEQ ID No. 40) and/or
EB11-f(SEQ ID No. 49) and EB11-r (SEQ ID No. 50)

6. Process according to one of the preceding claims, **characterized in that** the aberration of TIHO_EB 1 (SEQ ID No. 1) is mutTIHO_EB 1 (SEQ ID No. 11), the aberration of TIHO_EB 2 (SEQ ID No. 2) is mutTIHO_EB 2 (SEQ ID No. 12), the aberration of TIHO_EB 3 (SEQ ID No. 3) is mutTIHO_EB 3 (SEQ ID No. 13), the aberration of TIHO_EB 4 (SEQ ID No. 4) is mutTIHO_EB 4 (SEQ ID No. 14), the aberration of TIHO_EB 5 (SEQ ID No. 5) is mutTIHO_EB 5 (SEQ ID No. 15), the aberration of TIHO_EB 6 (SEQ ID No. 6) is mutTIHO_EB 6 (SEQ ID No. 16), the aberration of TIHO_EB 7 (SEQ ID No. 7) is mutTIHO_EB 7 (SEQ ID No. 17), the aberration of TIHO_EB 8 (SEQ ID No. 8) is mutTIHO_EB 8 (SEQ ID No. 18), the aberration of TIHO_EB 9 (SEQ ID No. 9) is mutTIHO_EB 9 (SEQ ID No. 19), the aberration of TIHO_EB 10 (SEQ ID No. 10) is mutTIHO_EB 10 (SEQ ID No. 20), and the aberration of TIHO_EB 11 (SEQ ID No. 47) is mutTIHO_EB 10 (SEQ ID No. 48).

7. Process according to one of the preceding claims, **characterized by** selecting a non-human individual for breeding.

8. Use of a DNA section comprising a DNA section which is amplified from total DNA of an individual in a process according to one of the preceding claims, wherein the DNA section is amplified by PCR with one of the following primer pairs:
EB1-f (SEQ ID No. 21) and EB1-r (SEQ ID No. 22),
EB2-f (SEQ ID No. 23) and EB2-r (SEQ ID No. 24),
EB3-f (SEQ ID No. 25) and EB3-r (SEQ ID No. 26),
EB4-f (SEQ ID No. 27) and EB4-r (SEQ ID No. 28),
EB5-f (SEQ ID No. 29) and EB5-r (SEQ ID No. 30),
EB6-f (SEQ ID No. 31) and EB6-r (SEQ ID No. 32),
EB7-f (SEQ ID No. 33) and EB7-r (SEQ ID No. 34),
EB8-f (SEQ ID No. 35) and EB8-r (SEQ ID No. 36),
EB9-f (SEQ ID No. 37) and EB9-r (SEQ ID No. 38),
EB10-f (SEQ ID No. 39) and EB10-r (SEQ ID No. 40), and/or
EB11-f (SEQ ID No. 49) and EB11-r (SEQ ID No. 50).

9. Use according to claim 8, **characterized in that** the DNA section comprises at least one of TIHO_EB 1 (SEQ ID No. 1), TIHO_EB 2 (SEQ ID No. 2), TIHO_EB 3 (SEQ ID No. 3), TIHO_EB 4 (SEQ ID No. 4), TIHO_EB 5 (SEQ ID No. 5), TIHO_EB 6 (SEQ ID No. 6), TIHO_EB 7 (SEQ ID No. 7), TIHO_EB 8 (SEQ ID No. 8), TIHO_EB 9 (SEQ ID No. 9), TIHO_EB 10 (SEQ ID No. 10), TIHO_EB 11 (SEQ ID No. 47) and/or mutTIHO_EB 1 (SEQ ID No. 11), mutTIHO_EB 2 (SEQ ID No. 12), mutTIHO_EB 3 (SEQ ID No. 13), mutTIHO_EB 4 (SEQ ID No. 14), mutTIHO_EB 5 (SEQ ID No. 15), mutTIHO_EB 6 (SEQ ID No. 16), mutTIHO_EB 7 (SEQ ID No. 17), mutTIHO_EB 8 (SEQ ID No. 18), mutTIHO_EB 9 (SEQ ID No. 19), mutTIHO_EB 10 (SEQ ID No. 20), and mutTIHO_EB 11 (SEQ ID No. 48).

10. Nucleic acid molecule comprising a sequence of a genetic marker for use in the analysis of the genetic disposition of an individual for JEB in a process according to one of claims 1 to 7, **characterized in that** the genetic marker comprises a sequence contained in the group consisting of TIHO_EB 1 (SEQ ID No. 1), TIHO_EB 2 (SEQ ID No. 2), TIHO_EB 3 (SEQ ID No. 3), TIHO_EB 4 (SEQ ID No. 4), TIHO_EB 5 (SEQ ID No. 5), TIHO_EB 6 (SEQ ID No. 6), TIHO_EB 7 (SEQ ID No. 7), TIHO_EB 8 (SEQ ID No. 8), TIHO_EB 9 (SEQ ID No. 9), TIHO EB 10 (SEQ ID No. 10), TIHO_EB 11 (SEQ ID No. 47) and/or mutTIHO_EB 1 (SEQ ID No. 11), mutTIHO_EB 2 (SEQ ID No. 12), mutTIHO_EB 3 (SEQ ID No. 13), mutTIHO_EB 4 (SEQ ID No. 14), mutTIHO_EB 5 (SEQ ID No. 15), mutTIHO_EB 6 (SEQ ID No. 16), mutTIHO_EB 7 (SEQ ID No. 17), mutTIHO_EB 8 (SEQ ID No. 18), mutTIHO_EB 9 (SEQ ID No. 19), mutTIHO_EB 10 (SEQ ID No. 20), mutTIHO_EB 11 (SEQ ID No. 48).

11. Nucleic acid molecule according to claim 10, **characterized in that** the nucleic acid molecule is present in multiple copies which are synthesized by PCR.

12. Oligonucleotide for use as a primer in a process according to one of claims 1 to 7 for the synthesis of an amplificate from total genomic DNA of an individual, selected from the group consisting of the pairs of primers
EB1-f (SEQ ID No. 21) and EB1-r (SEQ ID No. 22),
EB2-f (SEQ ID No. 23) and EB2-r (SEQ ID No. 24),
EB3-f (SEQ ID No. 25) and EB3-r (SEQ ID No. 26),
EB4-f (SEQ ID No. 27) and EB4-r (SEQ ID No. 28),
EB5-f (SEQ ID No. 29) and EB5-r (SEQ ID No. 30),
EB6-f (SEQ ID No. 31) and EB6-r (SEQ ID No. 32),
EB7-f (SEQ ID No. 33) and EB7-r (SEQ ID No. 34),
EB8-f (SEQ ID No. 35) and EB8-r (SEQ ID No. 36),
EB9-f (SEQ ID No. 37) and EB9-r (SEQ ID No. 38),
EB10-f (SEQ ID No. 39) and EB10-r (SEQ ID No. 40), and/or EB11-f (SEQ ID No. 49) and EB11-r (SEQ ID No. 50).
